Europäisches Patentamt

European Patent Office

Office européen des brevets

(19)

(11) Publication number: **0 448 647 B1**

# EUROPEAN PATENT SPECIFICATION

(45) Date of publication of patent specification: **28.06.95**　(51) Int. Cl.6: **B01D  71/56**

(21) Application number: **90901977.0**

(22) Date of filing: **15.12.89**

(86) International application number:
**PCT/US89/05617**

(87) International publication number:
**WO 90/06806 (28.06.90 90/15)**

The file contains technical information submitted
after the application was filed and not included in
this specification

(54) **HEAT RESISTANT MICROPOROUS MATERIAL PRODUCTION AND PRODUCTS.**

(30) Priority: **16.12.88 US 285787**

(43) Date of publication of application:
**02.10.91 Bulletin  91/40**

(45) Publication of the grant of the patent:
**28.06.95 Bulletin  95/26**

(84) Designated Contracting States:
**AT BE CH DE ES FR GB IT LI LU NL SE**

(56) References cited:
**WO-A-88/05686**
**US-A- 4 340 480**
**US-A- 4 617 235**
**US-A- 4 788 226**
**US-A- 4 814 356**

(73) Proprietor: **MICRON SEPERATIONS, INC.**
**135 Flanders Road**
**Westbough, MA 01581 (US)**

(72) Inventor: **JOHNSON, James, S.**
**15 Hemlock Lane**
**Acton, MA 01720 (US)**
Inventor: **CARTER, Edward, T.**
**12 Eureka Terrace**
**Worcester, MA 01603 (US)**

(74) Representative: **Dubois-Chabert, Guy**
**Société de Protection des Inventions**
**25, rue de Ponthieu**
**F-75008 Paris (FR)**

EP 0 448 647 B1

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

## Description

FIELD OF THE INVENTION

This invention relates generally to heat resistant microporous materials produced from a polyamide resin which is suitable for use in such applications as filtering bacteria from solutions, binding DNA in hybridization research and chromatography.

BACKGROUND OF THE INVENTION

Microporous materials have varied applications in the fields of separations, analytics and absorption. Filtration is the most fully developed of these arts and provides some technical background for the instant invention.

Microporous materials are characterized by their filtrate permeability (flow rate), minimum particulate retention size (pore size), continued permeability as particulates are collected, (clogging resistance), strength, dimensional stability, and wettability.

The flow rate of a porous material is a measurement of volume of fluid passage per unit time for a given depth and area of the material at a known differential pressure. Flow rate is influenced by the size, internal physical structure and distribution of the pores; for a given material area, depth and maximum pore size (measured by bubble point or particulate challenge), lower flow rates indicate fewer pores, a greater portion of smaller sized pores, or both.

Pore size may be characterized by the bubble point, [1] a measurement of the gas pressure required to remove a liquid from the pores of a saturated material. Bubble point measurements are typically reported for the removal of water by air, a standard of comparison now recognized in the microfiltration industry. If, for example, a filter formed from microporous material exhibits a bubble point of 41 kPa (6 psi) and a flow rate of 13 cc/min cm2 at 69 kPa (10 psi), then the relationship of flow rate and bubble point is not good relative to commercial filters. This bubble point is indicative of a pore size of around 5.0 $\mu$m, whereas the flow rate is indicative of a 0.2 $\mu$m pore size. This condition produces a membrane of poor filtration performance, having a comparatively low flow rate for the maximum pore size of 5 $\mu$m.

Pore size can also be measured by particulate challenge, the process of checking a material's particulate retention for progressively smaller particulates of known dimensions. Gradients of particulate size are commercially available and detection of particulate passage through the material is possible by a variety of analytical techniques, such as light scattering analysis, turbidity analysis, or subsequent smaller pore filtration and particle counts. Particulate challenge is generally more accurate and convenient for pore sizes greater than 50 $\mu$m than are bubble point determinations.

Clogging resistance is measured by passage or absorption of successive aliquots of a solution containing particulates retained by the material, then comparing the passage or absorption time of the successive aliquots. Commercial scale filtration of microscopic particulates has long been complicated by filters formed from materials which rapidly clog, and thus require frequent cleaning or replacement, thereby limiting filtration capacity.

Strength and dimensional stability of porous materials determine the manufacturing tolerances required for successful formation of commercial grade products, the differential pressure which the materials can withstand, as well as resistance to mechanical tear. For example, in filtration applications swelling and growth of unsupported nylon materials due to solvent absorption can cause mechanical problems.

Wettability is a material's propensity to absorb a particular solvent, and can be measured by the weight percentage of solvent absorbed at saturation, or by the time required for dry materials to reach saturation upon direct contact with the solvent ("wet out time"). Because some porous nylon materials are reported to exhibit reduced wettability at or near their melting points, See Pall, US-A- 4,340,479, saturation capacity and saturation time are generally considered as specific to temperature ranges. While the performance of porous materials with low wettability can be improved by pre-wetting with low surface tension solvents, followed by flushing with the target solvent immediately prior to use, pre-wetting is not only expensive due to cost of the additional solvent, but also due to the additional steps required to use the material for its intended purpose.

Because porous materials are often formed in sheets, fabrication of commercial products often requires sealing multiple surfaces of the material in order to form desirable configurations, for example, filtration

1 American Society for Testing and Materials, Standard F316; and Brock, Thomas D., Membrane Filtration: A User's Guide and Reference Manual, Science Tech, Inc., Madison, Wi., 1983.

cartridges. Seals formed by heating and then placing in contact the affected surfaces are inexpensive and do not require the use of adhesives which might contaminate the filtrate. Thermally formed seals however often reduce the wettability of porous nylon materials.

Several filters have been synthesized from nylon polymer materials. Paine's US-A-3,408,315 describes a membrane made by first dissolving nylon polymer in a solvent or solvents, forming a base solution and then adding other miscible reagent mixtures to the base solution. A thickening agent such as Cab-O-Sil fumed silica is added to increase the viscosity of the solution to the range of 500-1000 mPa•s (centipoise) (Brookfield). Paine's nylon terpolymer solution is then metered onto a belt and the solvents are evaporated, causing precipitation of the nylon into a thin film porous structure. This is known as an air casting process because air is used as the fluid to carry away the solvents from the film of polymer solution.

Paine membranes have limited utility due to their solubility in alcohol and many other solvents. Film membranes produced by this casting method have relatively weak tear and puncture resistance and undergo problematic dimensional changes when wetted with water or on drying after being wet with water.

Marinaccio et al.'s US-A- 3,876,738 describes a process for producing alcohol insoluble microporous membranes of nylon polymers using a wet casting process in which a liquid in contact with the cast film serves to remove the solvent from the polymer containing solution. The Marinaccio process involves metering a controlled thickness of polymer solution onto a drum which is partially longitudinally immersed in a bath containing a non-solvent for the polymer. As the drum rotates the cast film is immersed into the bath. As the solvent is extracted by the bath, the polymer precipitates as a film on the drum; removal of the film and subsequent processing are determined by the intended end use of the film.

Pore formation in Marinaccio's method is dependent upon polymer concentration, the solvent system used to make the polymer solution, the age of the polymer solution, composition of the solvent extraction bath, bath temperature and additives to the mix or the bath. Marinaccio explains that the pore size would increase slightly with polymer concentration because of the increasing aggregation tendency at higher concentrations because the more polymer in solution, the longer the chains of these polymers, and hence the larger spherical shape they will form when precipitated. These aggregates are Marinaccio's means of controlling pore sizes and these aggregation tendencies are modified by using various ratios of non-solvent to solvent, thereby changing the overall solvent power of the solution.

Marinaccio's film membranes suffer from relatively low tear resistance and strength, and dimensional instability. Because nylon polymers readily absorb up to eight weight percent water, the films swell and grow in size, causing problems in filtration and other uses.

Pall's US-A-4,340,479 claims a skinless, microporous, hydrophilic polyamide membrane produced from alcohol insoluble, hydrophobic nylon polymers, a phenomenon which he claims only occurs with "... ratios of $CH_2$ (methylene) to NHCO (amide) within the range from about 5:1 to 7:1." (Column 8, line 24), thus teaching away from the use of polyamide polymers with a $CH_2$ to NHCO ratio outside the range of 5:1 to 7:1 when synthesizing hydrophilic membranes.

Pall's method begins with nylon 66 resin dissolved in a solvent by a mixing regimen. To this starting solution another solution or non-solvent blend is added to create a visible precipitate of polymer, a necessary stage which results in what Pall terms "nucleation" or a "state of nucleation". The precipitate is then totally or partially redissolved to form a casting solution which is formed into a thin film on a substrate and with minimum delay is immersed in a bath which is comprised of a nonsolvent for the polymer and a solvent for the starting solvent. Pall states that many factors influence the nucleation state of the casting solution and hence the final filter properties. These include the temperature of the starting polymer and solvent; the rate and intensity of mixing by which the non-solvent mixture is added; and, the geometry of the mixing vessel.

Pall describes alcohol-insoluble polyamide resins as inherently "hydrophobic," and claims a method of producing from these resins membranes which are "hydrophilic" unless heated to near melting where they revert to the "hydrophobic" state. Because the near melting point temperatures used to effect thermal seals can result in a reversion of these membranes to a "hydrophobic" state, Pall membranes may not be wettable in the seal area with high surface tension solvents such as water and therefore difficult to test and problematical in application.

OBJECT AND SUMMARY OF THE INVENTION

The instant invention is a microporous material with pore sizes which range from about 0.01 $\mu$m to about 100 $\mu$m which retains its wettability over a range of temperatures sufficient to form thermal seals between adjacent portions of the material, and the process for synthesizing such materials. These seals are formed by a variety of mechanisms, which functionally define the particular temperatures at which the wettability of the material will not substantially degenerate over the period of time required to form a seal.

These porous materials are produced from nylon 46, a polyamide only recently made available commercially in the United States, synthesized by a polymerizing condensation reaction using diaminobutane and adipic acid as feed stocks. Nylon 46 is alcohol insoluble and has a $CH_2$ (methylene) to NHCO (amide) ratio of 4:1.

Relative to porous materials, and particularly in comparison to commercially available nylon filtration membranes, porous nylon 46 materials of this invention exhibit surprising clogging resistance and chemical resistance to a broad range of solvents and solutes. Users of filters and other devices incorporating porous nylon 46 materials of this invention, such as pleated filter cartridge elements in a filtration system, will realize increased throughput rates, longer cartridge life and more fluid filtered per cartridge with reduced down time to replace dirty, clogged filter elements, and an obvious cost reduction.

The wettability of nylon 46 microporous materials is substantially increased compared to other nylon products as demonstrated by the water absorption of nylon 46 as compared to nylon 66. At saturation nylon 46 absorbs 12 weight percent water (Allied Signal Product Bulletin 1987) whereas nylon 66 absorbs 8.5 weight percent. (DuPont Zytel Product Bulletin E 09134 8/78). This 40 percent increase in water absorption shows that nylon 46 has a higher affinity for water, hence is more wettable. In contrast with other nylon materials, the superior wettability of nylon 46 membranes enables direct contact saturation with solvents having higher surface tensions than water without using alcohol or some other fluid of low surface tension to prewet the material, thereby avoiding contamination by prewetting fluid and the resulting waste material produced when the wetting fluid is flushed from the material.

Another advantage of nylon 46 is increased temperature resistance resulting from the polymer's melting point of 290°C compared to 255°C for nylon 66.

The instant invention entails a microporous material and process parameters used to produce this material. The nylon 46 polymer is dissolved in a mixture of solvents and nonsolvents at a temperature and with sufficient agitation to completely dissolve the polymer without substantial polymer degradation. Solid materials are formed in a bath by precipitating the polymer solution in a controlled manner to produce a uniform pore structure. The precipitation bath is followed by a rinse tank which removes residual solvent from the material.

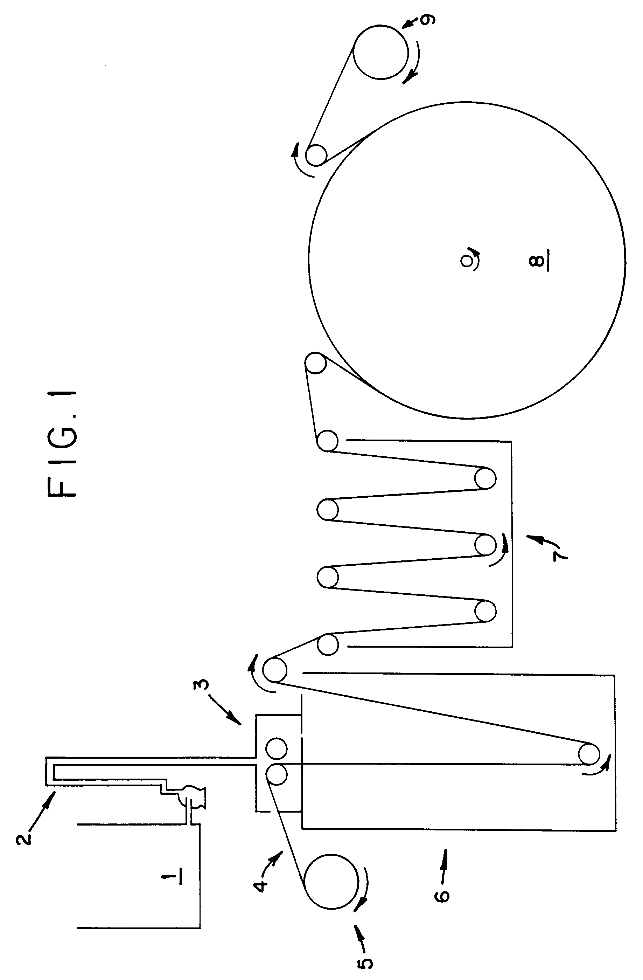

## BRIEF DESCRIPTION OF THE DRAWING

As shown in the drawing, a preferred embodiment of the process for producing sheets of microporous, hydrophilic filter from nylon 46 in accordance with the present invention employs a knife-film forming mechanism.

## DETAILED DESCRIPTION OF THE INVENTION

This invention uses nylon 46, a commercially new polyamide resin, to form a microporous, heat resistant, material with pore sizes ranging from about 0.01 to 100 $\mu$m, preferably from about 0.1 $\mu$m to about 20 $\mu$m. In a container the solvents and nonsolvents for the nylon 46 are blended, then nylon 46 mixed into the liquid until dissolved. The solvents are chosen from the group of aqueous bronsted acids, including but not limited to, hydrochloric acid, formic acid, phosphoric acid, and mixtures thereof. The nonsolvents are chosen from the group comprised of polar organic liquids which when mixed with the polymer and solvent result in the desired pore size of the filter, including but not limited to, methanol, ethanol, propanol, aqueous citric acid, water and mixtures thereof, with the preferred nonsolvent being water. Because greater proportions of nylon 46 polymer in the mix produce smaller pores in the resulting filter, a range of pore sizes from about 0.1 $\mu$m to about 20 $\mu$m can be produced from the range of about 11 - 16 weight percent nylon 46 polymer added to a mix of about 40 - 50 weight percent standard commercial reagent concentration ("reagent concentration") solvent and about 34 - 49 weight percent reagent concentration nonsolvent.

Nylon 46 is slowly added into the mixing solvents and nonsolvents at temperature range of about 25 - 80°C at a speed sufficient to prevent the polymer from clumping, but insufficient to cause overheating and polymer degradation. Within this range, higher temperatures cause dissolution to proceed more rapidly and the mix time to total dissolution can be decreased.

A variety of mixing devices for blending pellitized or powered solids with liquids to form viscous fluids are known in the art. The mix times necessary to dissolve the polymer range up to about 6 hours, depending upon the condition of the polymer; a finely powdered polymer will dissolve more quickly than an extruded, pelletized polymer.

After mixing is completed and the polymer is dissolved, the mix is filtered to remove any extraneous particles which would present problems in lacquer delivery or occlusions in the membrane.

Air bubbles created in the mixing step are then eliminated, preferably by letting the solution stand, thus preventing voids or defects in the material.

The solution is then cast into the shape of the desired material. Filter membranes are cast by dispersion into a uniformly thick film, preferentially upon a nonwoven web material. As previously noted, the relative proportions of nylon 46 and nylon 46 solvents and nonsolvents determine in part the pore size and density of the resulting material. Since higher solution temperatures result in somewhat larger pore sizes, temperature controls can be further used to manipulate the pore size of the material. The dispersion system can include temperature controls, preferentially a heat exchanger, to change the viscosity of the mixture as is necessary to obtain a smooth, even coating of the mixture. As the temperature of the mixture rises, and as these higher temperatures are maintained for longer periods of time, pore size is increased. This feature allows production flexibility because the solution's temperature can be manipulated to produce a range of pore sizes from a single batch of solution. The composition and process temperature control manipulations enable continuous production of the material with fixed or variable pore size and distribution from a single batch of nylon 46 solution.

A solution of nylon 46 lacquer (1) is pumped through a system of piping containing a heat exchanger (2) to the coating mechanism (3). The lacquer is dispersed on a supporting surface, preferably a nonwoven cloth web (4) to form a uniformly thick composite, preferably by drawing the cloth web from a roll thereof (5) through a knife box (3) which controls the thickness of the coating to the desired setting. The lacquer coated web then travels through an acid quench bath (6) causing precipitation of the polymer. The precipitated polymer coated web is then passed through a rinse tank (7) to remove residual solvents. A dryer (8) reduces the moisture content of the precipitated polymer coated web, resulting in the production of the microporous material. A wind up device (9) is used to roll the sheet of material onto a core for convenient storage.

The preferred dispersion system, a coating mechanism (3), is used to create a film of the solution, preferentially supported by a nonwoven fabric substrate. In the preferred embodiment a uniformly thick film of the mixture is assured by a knife box (3) having an adjustable gap which allows the final thickness of the membrane to be controlled. The cast mixture is then exposed to a bath comprised of both solvents and nonsolvents for nylon 46 in a range of compositions such that microporous nylon 46 polymer is precipitated in a uniform, consistent manner. Since smaller pore size materials result from higher proportions of solvents in the bath, a range of different pore sizes can be produced from a single batch of solution. The bath composition can range from about 0 - 60 weight percent hydrochloric, formic, citric or phosphoric acids, the percentage of stronger acids being chosen from the lower portion of this range. The remaining weight percentage is comprised of one or more of the previously recited nonsolvents. The bath concentrations are varied along with the lacquer composition to produce the desired material pore size.

Alternately, a filter plug for columnar applications can be cast from a cylindrical or other three dimensional mold, then exposed to the bath.

The nonsolvents are then extracted from the precipitated nylon 46 by a substance with higher affinity for the nonsolvents than nylon 46, preferably by passage through a rinse tank (7) utilizing a series of rollers to lead the membrane in a torturous contact with water. Extractables are materials which can leach out of the material when in contact with a fluid, and could lead to contamination of process fluids. The nylon 46 material produced by this invention is low in extractables, typically less than 0.1 percent by weight.

Excess moisture is removed from the filter, ideally by a hot air dryer (8) positioned to blow across the material when supported by a rotating drum. Filtered air heated with resistance heaters to a temperature in the range of 80 - 200°C can be used. The dry material is then rolled up on a core (9) for storage and subsequent use.

The porous materials produced according to this invention have a uniquely bimodal or "skinned" structure. More particularly, scanning election micrographs of the porous material produced according to the present invention show the interior of the material to have large interconnecting chambers that do not appear at the surface of the material.

This property of the materials produced according to the present invention can be further characterized by measuring the relative sizes of the pores in the surface and in the interior of the membrane. It has been found that the interior pores of the material are from about two to about ten times the diameter of the largest pores in the membrane surfaces. In addition, it has been found that membranes produced from the materials of the present invention have a porosity greater than about 50 percent.

In contrast, the microporous nylon 66 membrane described in Pall is skinless, i.e. the membrane has pores extending from surface to surface that are substantially uniform in shape and size. Likewise, the microporous membrane described in US-A-4,788,226 (Curry), which is said to be formed either of polytetramethylene adipamide alone or in admixture with at least one other polyamide, is also said to be skinless.

Heat resistant microporous materials produced according to this invention can be fashioned into desirable configurations and products by thermal seals which do not impair the wettability of the material. The wettability of this material is not substantially impaired after seals are formed at temperatures maintained for periods of time necessary for the sealing mechanisms of thermoplasticity, fusion, welding, glass transitions, solid/liquid phase transformation, softening point or when steam sterilized or exposed to temperatures of 126 - 135°C for about 45 minutes.

These materials are useful for filtration of bacteria from solutions, for separations required in biotechnical processes and for certain medical procedures, for absorption of contaminates and for separation techniques such as chromotography.

### Example 1: **Unsupported Material**

A solution of 14 weight percent nylon 46 polymer, 5.45 weight percent hydrochloric acid, 40.5 weight percent citric acid and 40.05 weight percent water was mixed at 30 degrees centigrade for 5 hours. The mix was cast onto a glass plate then immersed into a bath containing 29 weight percent citric acid and 71 weight percent water. The film produced was rinsed with water and removed from the glass plate. The material's mean clean water flow rate was 28 cm$^3$/min cm2 at 91 kPa (27" mercury) differential pressure with a bubble point of 165 kPa (24 psi), indicating a pore size of about 0.5 $\mu$m, at a thickness of 110 $\mu$m.

### Example 2: **Large Pore Size Supported Material**

A solution of 12 weight percent nylon 46 polymer, 5.7 weight percent hydrochloric acid, 41.2 weight percent citric acid and 41.1 weight percent water was mixed at 35 degrees centigrade at a mixer speed of 1300 rpm for 4 hours. This solution was cast onto a non-woven polyester substrate in a bath of approximately 25 weight percent citric acid, 1 weight percent hydrochloric acid and 74 weight percent water. The mix produced a sheet of material 70-80 $\mu$m in thickness with a mean clean water flow rate of 90 cm$^3$/min cm2 at 91 kPa (27" mercury) differential pressure and a bubble point of 83-103 kPa (12 to 15 psi), a pore size of approximately 0.8 $\mu$m.

### Example 3: **Small Pore Size Supported Material**

A solution of 14 weight percent nylon 46 polymer, 5.7 weight percent hydrochloric acid, 40.5 weight percent citric acid and 39.8 weight percent water was mixed at 35 degrees centigrade at a mixer speed of 1300 rpm for 4 hours. This solution was cast onto a non-woven polyester substrate then passed through a bath of approximately 30 weight percent citric acid, 2-3 weight percent hydrochloric acid and 67-68 weight percent water. This solution produced a filter of 70-80 $\mu$m in thickness with a mean clean water flow rate of 5.5cm$^3$/min cm2 at 91 kPa (27" mercury) differential pressure and a bubble point of 517-586 kPa (75-85 psi), a pore size of approximately 0.1 $\mu$m.

### Example 4: **Variable Pore Size Supported Material**

A solution of 14 weight percent nylon 46 polymer, 5.4 weight percent hydrochloric acid, 40.5 weight percent citric acid and 40.1 weight percent water was mixed at 35 degrees centigrade for 5 hours. This solution was cast onto a non-woven polyester substrate then passed through a bath of 32 weight percent citric acid, 2 weight percent hydrochloric acid and 66 weight percent water and produced a filter with a mean clean water flow rate of 2.5 cm$^3$/min cm2 at 91 kPa (27" mercury) differential pressure and a bubble point of around 690 kPa (100 psi), a pore size of less than 0.1 $\mu$m.

The heat exchanger and a different bath was then used to increase the pore size of the membrane produced from this solution batch. A solution at 39.5 degrees centigrade, and a bath of 22 weight percent citric acid, 1 weight percent hydrochloric acid and 77 weight percent water produced a membrane with a mean clean water flow rate of 37 cm$^3$/min cm2 at 91 kPa (27" mercury) differential pressure and a bubble point of 152-165 kPa (22-24 psi), indicating a pore size of approximately 0.5 $\mu$m.

Example 5: **Clogging Resistance**

An experiment was performed using ordinary tap water as the source of "dirty" fluid to determine clogging rates of nylon 46 filers versus commercially available nylon 66 filters. Samples of both nylon 46 and nylon 66 membranes were first tested for clean water flow rate and bubble point in order to identify membranes with similar pore size and flow rates. The nylon 46 membranes had a mean clean water flow rate of 16.5 $cm^3$/min $cm^2$ at 91 kPa (27" mercury) differential pressure while the nylon 66 membranes had a mean clean water flow rate of 19.8 $cm^3$/min $cm^2$ at the same pressure. The bubble points of the nylon 46 membranes were 276, 290 and 303 kPa (40, 42 and 44 psi) while the bubble points of the nylon 66 membrane were 296, 310 and 303 kPa (43, 45 and 46 psi). both membranes sets having pore sizes of approximately 0.3 $\mu$m.

Other 9.8 $cm^2$ disk samples of these membranes were then subjected to 50 ml of unfiltered tap water at a differential pressure of 91 kPa (27" mercury). For each 50 milliliter aliquot the time required to filter the fluid was recorded. Table 1 shows the sample of material tested, the filtration time (in seconds) for 50 ml of tap water, and the number of 50 ml aliquots filtered by the membrane sample. The tests were concluded when the filtration times increased approximately tenfold, an indication of filter clogging.

## TABLE 1

### Sample 1
### Nylon 66

**50ml aliquot**
**time (s)**

| | | | |
|---|---|---|---|
| 16.8 | 41.2 | 96.0 | 158 |

### Sample 2
### Nylon 66

**50ml aliquot**
**time (s)**

| | | | | |
|---|---|---|---|---|
| 16.3 | 27 | 59 | 92 | 149 |

### Sample 3
### Nylon 46

**50ml aliquot**
**time (s)**

| | | | | | | | | | | |
|---|---|---|---|---|---|---|---|---|---|---|
| 28 | 30 | 37 | 43 | 48 | 55 | 62 | 67 | 75 | 85 | 96 |

### Sample 4
### Nylon 46

**50ml aliquot**
**time (s)**

| | | | | | | | | | | |
|---|---|---|---|---|---|---|---|---|---|---|
| 25 | 32 | 41 | 48 | 57 | 63 | 70 | 77 | 85 | 92 | 100 |

For equivalent pore size material samples, flow rate decay, that is the time for filtration of each subsequent 50 ml portion of fluid, was found to be surprisingly lower for nylon 46 than nylon 66. A much larger volume of water was filtered by the nylon 46 representing a significant improvement in throughput ability of nylon 46 membranes and an increased resistance to clogging. In these samples throughput before clogging for the nylon 46 filters is more than double that of the commercially available nylon 66 filters.

A second experiment was performed to determine if the nylon 46 simply allowed more "dirt" to pass through without trapping it on the membrane. This experiment was carried out by filtering portions of 50 ml each, as above, of "dirty" fluid (tap water) through a nylon 46 membrane and using the filtrate, or collected, filtered water as the source of water for a nylon 66 membrane. (See Table 2)

## TABLE 2

### Sample 1 nylon 46
### Unfiltered tap water

50ml aliquot
time
(s)    25  32  41  48  57  53  70  77  85  92  100

### Sample 2 nylon 66
### Water pre filtered through sample 1 above

50ml aliquot
 time
(s)         14          14          14          14          14

### Sample 3 nylon 66
### Unfiltered tap water

50ml aliquot
 time
(s)         16.5        30          90          170

### Sample 4 nylon 46
### Water pre filtered through sample 3 above

50ml aliquot
time
(s)         19          19          19          19

As can be seen from the data, the nylon 66 membrane did not show any decay in flow time when the filtrate from a nylon 46 membrane was used in the flow decay test. This shows that the nylon 46 did retain the particles of dirt which would have clogged the nylon 66 membrane had the fluid not been pre filtered.

Using the filtrate from a nylon 66 membrane in the flow decay test for a nylon 46 membrane (Samples 3 and 4) no flow decay was observed. Since neither type of membrane clogged when using the filtrate from the other, this data shows that the two membranes performed equally well in removing particles from tap water. However, it is clear that nylon 46 membranes have significantly higher throughput before clogging than nylon 66 membranes of comparable particle retention and pore size distribution.

Example 6: **Wettability**

An experiment was performed to determine the wettability of nylon 46 membranes compared to nylon 66 membranes. Five sample filters, three of nylon 66 and two of nylon 46, were placed on the surface of various concentrations aqueous sodium chloride having surface tensions higher than pure water. Since increased surface tension reduces wettability of membranes, the time required for a 47 mm disk of membrane to completely wet out is indicative of filter wettability. The NaCl solutions provided a range of wet out times such that a wettability comparison of the sample membranes could be drawn.

TABLE 3

| WT. % NaCl | SURFACE TENSION mN/m (dynes/cm) | NYLON 66 0.6$\mu$m | NYLON 66 0.1$\mu$m | NYLON 66 1.2$\mu$m | NYLON 46 0.4$\mu$m | NYLON 4 0.2$\mu$m |
|---|---|---|---|---|---|---|
| 10 | 75.90 | instant | instant | instant | instant | instant |
| 15 | 77.68 | 2.3 s | 2.5 s | 2.5 s | instant | instant |
| 25 | 82.12 | no wet * | no wet * | 24 s | 0.5 s | 0.4 s |

* Filters did not wet out after 3 minutes

From Table 3 it can be seen that the nylon 46 membranes wet out in less time than nylon 66 membranes under conditions of higher surface tension. This demonstrates the advantage of nylon 46 over nylon 66 in wettability with fluids of higher surface tension, such as saline solutions.

**Claims**

1. A microporous material of nylon 46, characterized by having a plurality of pores in the exterior surface, and in the interior large interconnecting pores that do not appear at said exterior surface, the diameter of said large interconnecting pores being from about 2 to about 10 times larger than the diameter of the largest pores located in said exterior surface, said material substantially maintaining its original wet out time after heating to a temperature necessary to seal adjacent surfaces of the material.

2. The material of claim 1, characterized in that (a) the pores have an average diameter of about 0.01 to 100 $\mu$m as measured by bubble point or particulate challenge, and (b) the material is configured as a membrane having a fabric support.

3. The porous material of claims 1 and 2, characterized in that the pores have an average diameter of about 0.1 to 20 $\mu$m as measured by bubble point.

4. A liquid immersion casting process for producing a microporous material according to Claims 1-3 which substantially maintains its original wet out time after heating to a temperature necessary to seal adjacent surfaces of the material, characterized by the steps of :
   (a) preparing a mixture of (i) one or more nylon 46 solvents selected from the group comprised of aqueous bronsted acids and (ii) one or more nylon 46 nonsolvents selected from the group comprised of alcohols and polar organic compounds ;
   (b) dissolving nylon 46 into said mixture to form a solution ;
   (c) precipitating a portion of the nylon 46 from the solution ;
   (d) removing the nylon 46 nonsolvents from the precipitate to produce a microporous nylon 46 membrane having a plurality of pores in the exterior surface and in the interior large interconnecting pores that do not appear at the exterior surface, the diameter of said large interconnecting pores being from about 2 to 10 times larger than the diameter of the largest pores located on said exterior surface.

5. The process of claim 4, characterized in that :

(a) a 11-16 weight % nylon 46 solution is formed by dissolving it in a mixture containing 5-70 weight % nylon 46 solvents selected from the group hydrochloric acid, formic acid, phosphoric acid and/or mixtures thereof, and 14-84 weight % non solvents selected from the group water, aqueous citric acid, methanol, ethanol, propanol and/or mixtures thereof ;

(b) the nylon 46 is precipitated from the solution by contact with a solution containing about 0-60 weight % reagent concentration of citric acid or formic acid, or mixtures thereof, about 0-30 weight percent reagent concentration of hydrochloric, or phosphoric acids or mixtures thereof, and about 30-100 weight % nonsolvents selected from the group of water, methanol, ethanol, propanol and/or mixtures thereof ;

(c) the nylon 46 nonsolvents are removed from the precipitate by extraction with water ;

(d) the precipitate is dried with air heated in the range of 80-150 °C.

**Patentansprüche**

1. Mikroporöses Material aus Nylon 46, dadurch gekennzeichnet, daß es in der äußeren Oberfläche eine Vielzahl von Poren aufweist und im Innern große, miteinander verbundene Poren aufweist, die an der genannten äußeren Oberfläche nicht auftreten, wobei der Durchmesser der genannten großen, miteinander verbundenen Poren um etwa 2- bis etwa 10 mal größer ist als der Durchmesser der größten Poren, die in der äußeren Oberfläche angeordnet sind, wobei das Material nach dem Erhitzen auf eine Temperatur, die erforderlich ist, um benachbarte Oberflächen des Materials zu versiegeln, seine ursprüngliche Sättigungs-Benetzungszeit im wesentlichen beibehält.

2. Material nach Anspruch 1, dadurch gekennzeichnet, daß

a) die Poren einen durchschnittlichen Durchmesser von etwa 0,01 bis 100 $\mu$m, bestimmt mit dem Blasenbildungspunkt oder Teilchenbildungstest, aufweisen und

b) das Material als Membran mit einem Gewebeträger konfiguriert ist.

3. Poröses Material nach den Ansprüchen 1 und 2, dadurch gekennzeichnet, daß die Poren einen durchschnittlichen Durchmesser von etwa 0,1 bis 20 $\mu$m, gemessen mit dem Blasenbildungspunkt, aufweisen.

4. Flüssigkeitsimmersions-Gießverfahren zur Herstellung eines mikroporösen Materials nach den Ansprüchen 1 bis 3, das nach dem Erhitzen auf eine Temperatur, die zum Versiegeln benachbarter Oberflächen des Materials erforderlich ist, seine ursprüngliche Sättigungs-Benetzungszeit im wesentlichen beibehält, gekennzeichnet durch die folgenden Stufen:

a) Herstellung einer Mischung von (i) einem oder mehr Nylon 46-Lösungsmitteln, ausgewählt aus der Gruppe, der wäßrigen Bronsted-Säuren, und (ii) einem oder mehr Nylon 46-Nicht-Lösungsmitteln, ausgewählt aus der Gruppe der Alkohole und polaren organischen Verbindungen;

b) Auflösung von Nylon 46 in der genannten Mischung unter Bildung einer Lösung;

c) Ausfällung eines Teils des Nylons 46 aus der Lösung; und

d) Entfernung der Nylon 46-Nicht-Lösungsmittel aus dem Niederschlag unter Bildung einer mikroporösen Nylon 46-Membran, die in der äußeren Oberfläche eine Vielzahl von Poren aufweist und im Innern große, miteinander verbundene Poren aufweist, die an der äußeren Oberfläche nicht erscheinen, wobei der Durchmesser der großen, miteinander verbundenen Poren etwa 2- bis etwa 10 mal größer ist als der Durchmesser der größten Poren, die an der genannten äußeren Oberfläche angeordnet sind.

5. Verfahren nach Anspruch 4, dadurch gekennzeichnet, daß

a) eine 11 bis 16 gew.-%ige Nylon 46-Lösung gebildet wird durch Auflösen desselben in einer Mischung, die 5 bis 70 Gew.-% Nylon 46-Lösungsmittel, ausgewählt aus der Gruppe Chlorwasserstoffsäure, Ameisensäure, Phosphorsäure und/oder Mischungen davon, und 14 bis 84 Gew.-% Nicht-Lösungsmittel, ausgewählt aus der Gruppe Wasser, wäßrige Citronensäure, Methanol, Ethanol, Propanol und/oder Mischungen davon, enthält;

b) das Nylon 46 aus der Lösung ausgefällt wird durch Kontaktieren mit einer Lösung, die etwa 0 bis 60 Gew.-% Reagens-Konzentration an Citronensäure oder Ameisensäure oder Mischungen davon,

EP 0 448 647 B1

etwa 0 bis 30 Gew.-% Reagens-Konzentration an Chlorwasserstoffsäure oder Phosphorsäure oder Mischungen davon und etwa 30 bis 100 Gew.-% Nicht-Lösungsmittel, ausgewählt aus der Gruppe Wasser, Methanol, Ethanol, Propanol und/oder Mischungen davon, enthält;

c) die Nylon 46-Nicht-Lösungsmittel durch Extraktion mit Wasser aus dem Niederschlag entfernt werden; und

d) der Niederschlag mit Luft, die auf eine Temperatur in dem Bereich von 80 bis 150°C erhitzt ist, getrocknet wird.

**Revendications**

1. Matériau microporeux de nylon 46, caractérisé en ce qu'il possède une pluralité de pores dans la surface extérieure et à l'intérieur des grands pores d'interconnexion qui n'apparaissent pas au niveau de ladite surface extérieure, le diamètre desdits grands pores d'interconnexion étant d'environ 2 à environ 10 fois plus grand que le diamètre des pores les plus grands situés sur ladite surface extérieure, ledit matérieau conservant pratiquement son temps de mouillage initial après chauffage à une température nécessaire pour sceller des surfaces adjacentes du matérieau.

2. Matériau selon la revendication 1 , caractérisé en ce que (a) les pores ont un diamètre moyen d'environ 0,01 à 100 $\mu$m lorsqu'ils sont mesurés par le point de bulle ou l'épreuve des particules , et (b) le matériau est configuré comme une membrane comportant un support de tissu.

3. Matériau poreux selon les revendications 1 et 2, caractérisé en ce que les pores ont un diamètre moyen d'environ 0,1 à 20 $\mu$m lorsqu'ils sont mesurés par le point de bulle.

4. Procédé de coulée par immersion dans un liquide pour produire un matériau microporeux selon les revendications 1 à 3 qui conserve pratiquement son temps de mouillage initial après chauffage à une température nécessaire pour sceller des surfaces adjacentes du matériau, caractérisé par les étapes consistant à :
   (a) préparer un mélange de (i) un ou plusieurs solvants de nylon 46 choisis à partir du groupe comprenant des solutions aqueuses d'acides de Brönsted et (ii) un ou plusieurs produits non solvants de nylon 46 choisis dans le groupe constitué d'alcools et de composés organiques polaires ;
   (b) dissoudre le nylon 46 dans ledit mélange pour former une solution ;
   (c) précipiter une partie du nylon 46 à partir de la solution ;
   (d) éliminer les produits non solvants du nylon 46 du précipité pour produire une membrane microporeuse de nylon 46 comportant une pluralité de pores à la surface extérieure et, à l'intérieur, des grands pores d'interconnexion qui n'apparaissent pas à la surface extérieure , le diamètre desdits grands pores d'interconnexion étant d'environ 2 à 10 fois plus grand que le diamètre des pores les plus grands situés sur ladite surface extérieure.

5. Procédé selon la revendication 4, caractérisé en ce que :
   (a) une solution de nylon 46 de 11 à 16 % en poids est formée en le dissolvant dans un mélange contenant 5 à 70 % en poids de solvants de nylon 46 choisis dans le groupe constitué d'acide chlorhydrique, d'acide formique, d'acide phosphorique et/ou de leurs mélanges , et 14 à 84 % en poids de produits non solvants sélectionnés dans le groupe constitué d'eau , d'acide citrique étendu à l'eau, de méthanol, d'éthanol , de propanol et/ou de leurs mélanges;
   (b) le nylon 46 est précipité à partir de la solution par contact avec une solution contenant environ 0 à 60 % en poids d'une concentration de réactif d'acide citrique ou d'acide formique , ou de leurs mélanges , environ 0 à 30 % en poids d'une concentration de réactif d'acide chlorhydrique , ou d'acide phosphorique ou de leurs mélanges , et environ 30 à 100 % en poids de produits non solvants sélectionnés dans le groupe constitué par l'eau , le méthanol, l'éthanol , le propanol et/ou leurs mélanges;
   (c) les produits non solvants du nylon 46 sont éliminés du précipité par extraction à l'eau ;
   (d) le précipité est séché avec de l'air chauffé dans la gamme de 80 à 150°C.

12

FIG. 1